(19) Europäisches Patentamt
European Patent Office
Office européen des brevets

(11) Publication number: **0 492 409 A1**

(12) **EUROPEAN PATENT APPLICATION**

(21) Application number: **91121727.1**

(22) Date of filing: **18.12.91**

(51) Int. Cl.[5]: **C07K 15/28**, C12N 15/13, C12N 5/22, C12N 15/11, C12N 5/12, G01N 33/577, A61K 39/395

(30) Priority: **18.12.90 JP 417924/90**
**07.11.91 JP 350553/91**

(43) Date of publication of application:
**01.07.92 Bulletin 92/27**

(84) Designated Contracting States:
**AT BE CH DE DK ES FR GB GR IT LI LU MC NL SE**

(71) Applicant: **ISIHARA SANGYO KAISHA Ltd.**
**No. 3-22, Edobori 1-chome, Nishi-ku**
**Osaka(JP)**

(72) Inventor: **Mekada, Eisuke**
**654-1, Higashikushihara-cho**
**Kurume-shi, Fukuoka-ken(JP)**
Inventor: **Yamada, Nobutoshi, Ishihara Sangyo Kaisha Ltd.**
**Chuo Kenkyusho, 3-1, Nishi-Shibukawa 2-chome**
**Kusatsu-shi, Shiga-ken(JP)**
Inventor: **Tamai, Kiyoshi, Ishihara Sangyo Kaisha Ltd.**
**Chuo Kenkyusho, 3-1, Nishi-Shibukawa 2-chome**
**Kusatsu-shi, Shiga-ken(JP)**

(74) Representative: **Wächtershäuser, Günter, Dr.**
**Tal 29**
**W-8000 München 2(DE)**

(54) **Human monoclonal antibody.**

(57) A human monoclonal antibody with the class of immunoglobulin being IgM and with the type of L-chain being $\kappa$ and capable of recognizing a common antigen of ganglioside GM3 (N-acetylneuraminic acid), GT1b, GD3, GD1a and GQ1b present on the surface of a melanoma cell of MeWo, M12, M14 or G-361.

EP 0 492 409 A1

The present invention relates to a human monoclonal antibody which is useful as a diagnostic or therapeutic agent for melanoma and which is useful also as a reagent for the study of the role of a sugar chain in vivo and the relation between the sugar chain and the carcinogenetic mechanism, and a process for its production, a material for the production and a pharmaceutical composition.

A method for establishing a cell line for producing a uniform antibody having a specificity to a certain specific antigen determinant, has been reported by Milstein et al., Nature, Vol. 256, p. 495-497 (1975)), whereby it has been made possible to quantitatively or qualitatively analyze a very small amount of materials. On the other hand, many monoclonal antibodies specific to tumor cells have been produced by means of this technique for the detection of tumor antigens, and some of them have been found to be antibodies capable of recognizing a sugar chain of a lipid or a glycoprotein. For example, as a monoclonal antibody reactive with a human melanoma, an antibody reactive with a glycolipid of ganglioside GD3 or ganglioside GM3, has been obtained (J. Exp. Med., Vol. 155, p. 5392-5396 (1983) and Gann, Vol. 75, p. 418-426 (1984)). Such an antibody is useful for the diagnosis of carcinoma, and a therapeutic use thereof has been attempted. However, such an antibody is a foreign (mouse) protein to human and thus has a problem that it is likely to cause a serious side effect such as anaphylaxy or other allergy reactions when administrated into a human body.

To solve such a problem, it has been attempted to develop immunoglobulins derived from human i.e. not from foreign animals, and several immunoglobulins have already been reported as human monoclonal antibodies against melanoma (Proc. Natl. Acad. Sci., USA, Vol. 84, p. 2416-2420 (1987), ditto, Vol. 79, p. 7626-7633 (1982) and ditto, Vol. 80, p. 5392-5396 (1983)).

However, at present, these antibodies do not necessarily provide satisfactory results for all of melanoma patients. As a reason for such failure, there may be mentioned a change of the sugar chain structure on the tumor cell surface during the treatment, or it is impossible with a single antibody to cope with a variety of the sugar chain structures on the tumor cell surface, or even if the antibody has reacted to tumor cells, no effective cell-killing activities may be obtained.

To solve such problems, it is considered to provide a useful means to present an antibody which is capable of recognizing a common part of the variety of sugar chain structures expressed by melanoma. For this purpose, the present inventors have conducted extensive researches to obtain a human monoclonal antibody which is different from known human monoclonal antibodies and which is capable of recognizing a sugar chain structure on melanoma and has a complement dependent cytotoxicity (hereinafter referred to simply as CDC) against cells having the recognized antigen on their surface.

The present inventors have recovered lymphocytes from the peripheral blood of a melanoma patient, have transformed the lymphocytes with Epstein-Barr virus (hereinafter referred to simply as EBV) to obtain EBV-transformed human cell lines and have established from them a human cell line capable of producing an antibody having a CDC activity against melanoma. The reaction specificity of this antibody to various cells has been examined, and it has been found that this antibody shows a CDC activity against melanoma such as MeWo, M12, M14 or G-361, while it shows no substantial CDC activity against a human normal skin fibroblast, a human adenocarcinoma, a human neuroblastoma or a Vero cell and thus it is useful as a diagnostic or therapeutic agent for melanoma. Further, this antibody has been found to be a novel antibody, since the sugar chains recognized by this antibody are ganglioside GM3 (N-acetylneuraminic acid), GT1b, GD3, GD1a and GQ1b. The present invention has been accomplished on the basis of these discoveries.

Further, the present inventors have obtained mRNA from the above-mentioned EBV-transformed human cell line and have elucidated, by means of cDNA, the DNA sequence and the amino acid sequence of the V-region of each of H-chain and L-chain of this human monoclonal antibody, which shows an antigen specificity. Thus, the present invention has been accomplished also by gene manipulation.

The present invention provides a human monoclonal antibody with the class of immunoglobulin being IgM and with the type of L-chain being $\kappa$ and capable of recognizing a common antigen of ganglioside GM3 (N-acetylneuraminic acid), GT1b, GD3, GD1a and GQ1b present on the surface of a melanoma cell of MeWo, M12, M14 or G-361. The human monoclonal antibody shows a CDC activity against the melanoma cell having the common antigen on its surface, but shows no substantial CDC activity against a human normal skin fibroblast, a human adenocarcinoma, a human neuroblastoma or a Vero cell.

The present invention also provides a process for producing such a human monoclonal antibody, a human cell line transformed by EBV to produce such an antibody, and a diagnostic or therapeutic composition for melanoma, which contains such an antibody.

Further, the present invention provides an antibody gene encoding the above-mentioned human monoclonal antibody, a plasmid containing such a gene, a recombinant cell containing such a plasmid, a process for producing the antibody by means of such a recombinant cell, an antibody wherein the V-region of H-chain contains an amino acid sequence of the 1st Glu to the 117th Val as shown by SEQ ID NO:1 in the Sequence Listing, and the V-region of L-chain contains an amino acid sequence of the 1st Asp to the 114th Arg as shown by SEQ ID NO:2 in the Sequence Listing, and a diagnostic or therapeutic composition containing such an antibody.

Further, the present invention provides a gene encoding an amino acid sequence of the 1st Glu to the 117th Val as shown by SEQ ID NO:1, a gene containing a DNA sequence of the 123-125th GAG to the 471-473rd GTC as shown by SEQ ID NO:1, a gene encoding an amino acid sequence of the 1st Asp to the 114th Arg as shown by SEQ ID NO:2, and a gene containing a DNA sequence of the 66-68th GAG to the 405-407th CGA as shown by SEQ ID NO:2.

In the accompanying drawing, Figure 1 is a graph showing CDC activity (% lysis) against various cells by the antibody of the present invention.

The antibody of the present invention belongs to a class of IgM. The basic structure of IgM consists of a pentamer of a unit comprising two heavy chains (H-chains) and two light chains (L-chains) and one J-chain. The L-chain is a polypeptide having a molecular weight of about 25,000, and the H-chain is a polypeptide having a molecular weight of about 51,000. The J-chain is a polypeptide having a molecular weight of about 15,000 and has a sugar chain. The sequence of about 100 amino acids from the N-terminal of H-chain or L-chain is called a variable region (V-region) and has an antigen specificity. The sequences of about 400 amino acids and about 150 amino acids following the V-region are called constant regions (C-regions), where the amino acid sequences are constant at the levels of class and subclass.

Now, preparation and establishment of an EBV-transformed human cell line capable of producing the antibody of the present invention and preparation of the antibody will be described.

Firstly, from the peripheral blood of a melanoma patient, lymphocytes including B lymphocytes are recovered by e.g. usual specific gravity centrifugation, and subjected to transformation by EBV to obtain a cell line which is capable of constantly producing an antibody from the lymphocytes and which is immortalized indefinitely. EBV is known as a virus which is capable of transforming human normal B lymphocytes to a propagative cell line (Nature, Vol. 269, p. 410 (1973)). As such an EBV-containing material, B95-8 Marmoset lymphoblastoid cell line (Proc. Natl. Acad. Sci., USA, Vol. 87, p. 3333-3337 (1990)) may be mentioned. At the time of transfecting EBV, a propagation suppressive agent against T-cells, such as Cyclosporin A, is added to a lymphocyte suspension containing the desired B lymphocytes, followed by culturing, and at the time of the cell culturing, IL-6 inducing the propagation of EBV-transformed cells is added, followed by culturing to obtain an EBV-transformed human cell line (Science, Vol. 239, p. 502-504 (1988)).

Then, the culture solution of the cell line obtained by the above operation is subjected to screening based on the presence or absence of the CDC activity by means of melanoma cells (MeWo, M12, M14 or G-361) to establish the desired EBV-transformed human cell line. Such melanoma cells are specified as follows.

| | |
|---|---|
| MeWo cells: | obtained from Tokyo Metropolitan Clinical Medicine Research Center |
| M12 cells: | obtained from Seitai Bogyo Igaku Kenkyujo of Kyushu University |
| M14 cells: | obtained from Seitai Bogyo Igaku Kenkyujo of Kyushu University |
| G-361 cells: | ATCC No. CRL1424 (Deposition No. at American Type Culture Collection (ATCC)) |

Further, the EBV-transformed human cell line obtained by the above operation is cultured to produce the desired antibody. Purification of the antibody in the culture solution can be conducted by a proper combination of conventional biochemical and immunochemical procedures, such as ammonium sulfate precipitation, gel filtration, ion exchange chromatography, affinity chromatography, etc.

The purified antibody thus obtained is homogeneous and has a high titer, and thus it is suitable for use as a biochemical formulation. For example, after filtrating sterilization by e.g. a membrane filter, it may be used by itself, or it may be mixed with a pharmacologically acceptable carrier, excipient or diluent in a diagnostically or therapeutically sufficient amount of the antibody.

As a therapeutic composition, it may be formulated, for example, into an injection drug, and its effective dose depends on e.g. the degree of the disease, the age and the body weight of the patient and the manner of administration, and will be determined by a test for a proper amount conducted by a competent doctor. However, the dose is usually within a range of from about 10 ng to about 100 mg/kg of the body weight.

Further, the antibody of the present invention shows specificity to various melanoma cells. Therefore, it can be used as an immunodiagnostic agent by labeling the antibody with an enzyme or with a radioisotope.

Now, the antibody gene of the present invention will be described with reference to Examples. For the gene manipulation, conventional methods and techniques disclosed in many references may be employed. Some of such references will be listed below.

T. Maniatis et al: Molecular Cloning, Cold Spring Harbor Laboratory (1982), R. Wu et al: Methods in Enzymology, 100 and 101 (1983), R. Wu et al: Methods in Enzymology, 153, 154 and 155 (1987), F. M. Ausubel et al: Current Protocols in Molecular Biology, John Wiley & Sons, Inc.

With respect to the antibody gene of the present invention, a cDNA library is prepared by means of mRNA derived from the EBV-transformed human cell line capable of producing the human monoclonal antibody, then a plasmid containing cDNA encoding the desired human monoclonal antibody is isolated from the library, whereupon the DNA sequence of the antibody gene is determined. Such methods and operations are conducted in accordance with conventional manners as will be described in Examples 2 and 3. The mRNA will be obtained by culturing the above-mentioned EBV-transformed human cells, and subjecting the cells to lysis with a predetermined guanidinium isothiocyanate solution, followed by extraction. Using this mRNA as a template, cDNA is prepared and inserted to a suitable vector to obtain a cDNA library, and plasmid DNA containing such cDNA is fixed on a nylon filter. With respect to the C-region of the H-chain of human IgM, its DNA sequence and amino acid sequence are reported by T. H. Rabbitts, A. Foster, C. P. Milstein in Nucleic Acids Res., Vol. 9, p. 4509-4524 (1981); with respect to the C-region of the L-chain, the DNA sequence and amino sequence are reported by P. A. Hieter, E. E. Max, J.G. Seidman, J.V. Maizel Jr., P. Leder in Cell, Vol. 22, p. 197-207 (1980); and with respect to the J-chain, the DNA sequence and amino sequence are reported by J. E. Mole, A. S. Bhown, J. C. Bennett in Biochemistry, Vol. 16, p. 3507-3513 (1977). Oligonucleotides corresponding to parts of their domains are chemically synthesized. Using such chemically synthesized oligonucleotides as probes, colonies containing plasmid DNA reactive with such probes are isolated to obtain cell lines. From such cell lines, plasmid DNA fragments having cDNA of the H-chain and L-chain of the antibody are taken, and the DNA sequences of the cDNA portions are determined.

The gene of the antibody of the present invention is such that the V-region of H-chain has a DNA sequence encoding an amino acid sequence of the 1st Glu to the 117th Val as shown by SEQ ID NO:1 in the Sequence Listing, and the V-region of L-chain has a DNA sequence encoding an amino acid sequence of the 1st Asp to the 114th Arg as shown by SEC ID NO:2. Further, the V-region of H-chain has a DNA sequence of the 123-125th GAG to the 471-473rd GTC as shown by SEQ ID NO:1, and the V-region of L-chain has a DNA sequence of the 66-68th GAC to the 405-407th CGA as shown by SEQ ID NO:2.

On the other hand, with respect to the antibody gene of the present invention, the DNA sequence and the amino acid sequence have been made clear. Therefore, the antibody gene of the present invention can readily be prepared by a method and operation commonly employed in the chemical synthesis of a gene. For example, genes containing, respectively, a suitable DNA sequence (upper strand) having a predetermined amino acid sequence as shown by SED ID NO:1 or 2 and a complementary DNA sequence (lower strand), can be prepared by a method wherein each of such genes is chemically synthesized as divided into a plurality of oligonucleotides, and blocks of such synthesized oligonucleotides are then sequentially appropriately ligated. As the method for synthesizing such oligonucleotides, a diester method (H. G. Khorana: Some Recent Developments in Chemistry of Phosphate Esters of Biological Interest, John Wiley and Sons, Inc., New York (1961)), a triester method (R. L. Letsinger et al: J. Am. Chem. Soc., Vol. 89, p. 4801 (1967)) and a phosphite method (M. D. Matteucci et al: Tetrahedron Lett., Vol. 21, p. 719 (1980)) may be mentioned. However, in view of the operation efficiency, it is preferred to employ a phosphite method using a completely automated DNA synthesizer.

Synthesized oligonucleotides are then purified by a conventional purification method, such as high performance liquid chromatography using a reversed phase chromatography column, or electrophoresis using polyacrylamide gel. Thereafter, the oligonucleotides are phosphorylated by means of, e.g., T4 polynucleotide kinase, then annealed and ligated by means of T4 DNA ligase. Here, the oligonucleotides are divided into several blocks and sequentially ligated so that the antibody gene sequence will eventually be obtained, followed by digestion with restriction endonucleases or polishing (make blunt-end) with T4 DNA polymerase, and the resulting DNA fragments are then purified by electrophoresis. The obtained DNA fragments are inserted into plasmid vectors such as pUC8, pUC9, pUC18 and pUC19 (J. Messing et al., Gene, Vol. 19, p. 259 (1982)), and the inserted plasmid vectors are introduced into competent cells for cloning in accordance with a usual method. From the obtained clones, plasmid DNAs are extracted and purified by a usual method, and they are examined to see whether or not the nucleotide sequences of the DNA fragments inserted into the vectors agree with the desired gene sequence. The respective sections of the antibody gene thus obtained, are then cut out from the plasmid vectors containing them, by means of restriction endonucleases, then ligated and inserted again into the above vector to obtain a plasmid vector having the desired full length of the antibody gene. The plasmid vector thus obtained is digested by restriction endonucleases and separated and purified by gel electrophoresis to obtain the desired antibody gene.

Then, the antibody gene of the present invention is appropriately inserted into an expression vector to clone an antibody expression vector. The expression vector has a promoter region and an enhancer sequence upstream of the translation initiation codon (ATG) and a poly A signal region downstream of the translation termination codon (TAA, TGA or TAG). To insert the antibody gene to the expression vector, the genes for the predetermined H-chain and L-chain, and if necessary, the J-chain, as the antibody gene, may be inserted into a single plasmid, or may be inserted to separate plasmids. As the plasmid, pRc/CMV, pRc/RSV or the like may be employed.

The expression vector can be introduced into host cells by a conventional method, and the antibody can be produced by culturing the transformed host cells. As such host cells, Escherichia coli, yeast or human or other mammal cells, may be employed, and plasmids may also be selected for application to such host cells. Further, if antibody non-producing type human or other mammal cells having a J-chain gene are used as host cells, the antibody of the present invention may be produced by introducing an expression vector containing only genes of H-chain and L-chain of the present invention into the host cells.

By culturing the transformed host cells, the desired antibody is produced and accumulated in the culture, and the accumulated antibody is then separated. When animal cells are used as host cells, they can be cultured in accordance with a conventional method usually at 37° C for a few days under an atmosphere of 5% $CO_2$.

The antibody of the present invention can be obtained usually by conducting centrifugation after culturing to recover the culture solution and to remove the cells. The antibody thus obtained can be purified by a combination of conventional separation and purification methods for proteins such as filtration by a filter, affinity chromatography, anion or cation exchange chromatography, etc. The antibody thus obtained can be formulated and used as a diagnostic or therapeutic composition, as in the case of the above-described antibody produced by culturing the EBV-transformed human cell line.

The antibody of the present invention has low antigenicity to a human body since it is derived from human and thus is useful as a therapeutic composition for melanoma.

Now, the present invention will be described in further detail with reference to Examples. However, it should be understood that the present invention is by no means restricted to such specific Examples.

EXAMPLE 1

(1) Preparation and establishment of EBV-transformed human cell line

From the peripheral blood of a melanoma patient, lymphocytes containing desired B-lymphocytes were recovered by specific gravity centrifugation using Ficoll-Hypaque as a density gradient solution. To from about 0.5 to 5 $\times$ $10^7$ lymphocytes thereby obtained, a suitable amount of the supernatant of the culture of B95-8 marmoset lymphoblastoid cell line was added and transfected at 37° C for 3 to 4 hours under gentle shaking. Then, the lymphocytes were suspended in a 20% fetal calf serum-containing RPMI 1640 culture medium (hereinafter referred to simply as a FCS-RPMI culture medium), and Cyclosporin A was added thereto to a concentration of 2 $\mu$g/ml. At the same time, IL-6 capable of inducing the propagation of EBV-transformed cells was added thereto to a concentration of 1.9 $\times$ $10^3$ U/ml, followed by culturing for from 2 to 3 weeks on a cell culture plate with 96 wells (in an incubator at 37° C, 5% $CO_2$) to obtain an EBV-transformed human cell line.

The culture supernatant of the cell line obtained by the above operation, was subjected to screening based on the presence or absence of the CDC activity, followed by cloning by limiting dilution to establish an EBV-transformed cell line AKH11.

The measurement of the CDC activity was conducted as follows:

1.5 $\times$ $10^6$ melanoma cells (MeWo, M12, M14 or G-361) were added to a 5% FCS-RPMI culture medium (0.3 ml), and $^{51}$Cr-labeled sodium chromate was added thereto to a concentration of 0.5 mCi/ml, followed by incubation at 37° C for one hour. The respective melanoma cells used were as specified above. $^{51}$Cr-incorporated cells were washed four times with a Hank's solution and again suspended in a 5% FCS-RPMI culture medium (2 ml). To 0.1 ml of this cell suspension, 0.1 ml of the diluted solution of 300, 100, 30, 10 and 3 times dilution of the culture supernatant of the above transformed cell line was added and reacted at 37° C for 30 minutes, followed by centrifugation (5,000 rpm, five minutes), whereupon 0.15 ml of the supernatant was removed. To the remaining 0.05 ml fraction containing cells, an equal amount of a rabbit complement serum was mixed and reacted at 37° C for 45 minutes, followed by centrifugation (5,000 rpm, five minutes). The radioactivities (cpm) of 0.05 ml of this supernatant (A) and the remaining fraction (B) containing cells were measured by a $\gamma$-counter, whereupon the CDC activities (% lysis) against the respective melanoma cells, of the antibody at the respective concentrations in the supernatant of the culture of the EBV-transformed cell line were calculated as follows. The results are shown in Figure 1.

% lysis = [(A - Ac) × 2/(A + B) - Ac × 2] × 100

where Ac represents the background radioactivity of the control reacted only with the complement serum.

Using a human normal skin fibroblast, a human adenocarcinoma (SW-13), a human neuroblastoma (IMR-32)-, and Vero cells instead of the melanoma cells, the respective radioactivities were measured in the same manner as described above, and the CDC activities (% lysis) were calculated. The cells used were those specified as follows.

| | |
|---|---|
| Human normal skin fibroblast: | obtained from Cell Engineering Center of Osaka University |
| SW-13 cells: | ATCC No. CCL105 (Deposition No. at ATCC) |
| IMR-32 cells: | ATCC No. CCL127 (Deposition No. at ATCC) |
| Vero cells: | ATCC No. CCL81 (Deposition No. at ATCC) |

In Figure 1, (a) represents the results with MeWo, (b) represents the results with M12, (c) represents the results with M14, (d) represents the results with G-361, (e) represents the results with the human normal skin fibroblast, (f) represents the results with SW-13, (g) represents the results with IMR-32, and (h) represents the results with Vero cells.

From these results, it is evident that the antibody of the present invention is capable of specifically recognizing melanoma cells and thus can be used as a diagnostic agent for melanoma. Further, it reacts with melanoma cells to show a CDC activity and thus can be used as a therapeutic agent for melanoma.

(2) Preparation and identification of a monoclonal antibody capable of producing EBV-transformed cell line AKH11

The EBV-transformed cell line AKH11 established in the above step (1) was cultured for one week in a 20% FCS-RPMI culture medium using a Petri dish having a diameter of 10 cm (in an incubator at 37°C, 5% $CO_2$). This culture solution was subjected to centrifugation (1,000 rpm, 5 minutes) to obtain a culture supernatant containing the desired antibody.

Identification of the L-chain type and the class of immunoglobulin H-chain of the antibody of the present invention will now be described.

0.5 ml of a diluted solution (diluted to from 1 to 100 times) of the above-mentioned culture supernatant, was added to 0.15 ml of CNBr-activated Sepharose 4B (tradename, manufactured by Pharmacia) having goat anti-human IgA antibody (No. 0201-0021, manufactured by Cappel), goat anti-human IgG antibody (No. 0201-0121, manufactured by Cappel), goat anti-human IgM antibody (No. 0201-0201, manufactured by Cappel), goat anti-human Ig-$\kappa$ antibody (No. 0201-0241, manufactured by Cappel) or goat anti-human Ig-λ antibody (No. 0201-0281, manufactured by Cappel) coupled thereto, and reacted at 4°C for 16 to 18 hours for absorption. Each sample was subjected to centrifugation (1,000 rpm, 5 minutes), whereupon with respect to each supernatant, the CDC activity was measured using MeWo as target cells. As a result, the antibody of the present invention was absorbed specifically only by the goat anti-human IgM antibody and the goat anti-human Ig-$\kappa$ antibody. Thus, the antibody of the present invention was found to be such that the class of immunoglobulin H-chain was IgM and the L-chain type was $\kappa$.

Identification of the sugar chain antigen which the antibody of the present invention recognizes, will be described.

Using various glycolipids as antigens, the sugar chain antigens which the antibody of the present invention recognizes, were identified by an enzyme immunoassay as described below.

Firstly, various glycolipids were adsorbed on a microplate with 96 wells in an amount of 0.5 µg/well. Then, non-specific bonds were blocked by a 0.1% bovine serum albumin-containing 50 mM tris buffered physiologic saline (pH7.2, 4°C, 18 hours). Then, the culture supernatant of AXH11 cells diluted four times, was added in an amount of 50 µl/well and reacted at 37°C for one hour. Further, a peroxidase conjugated goat anti-human IgM antibody (No. 3601-0201, manufactured by Cappel) diluted 200 times, was added in an amount of 50 µl/well and reacted at 37°C for one hour. A substrate buffered solution (0.1 M citric acid, 0.2 M sodium phosphate dibasic and 0.005% hydrogen peroxide) containing 0.4 mg/ml of o-phenylenediamine as enzyme substrate was added in an amount of 100 µl/well and reacted, whereupon the absorbance at 492 nm was measured to examine the reactions of the antibody to various glycolipids.

As a result, it was found that the antibody of the present invention reacted with ganglioside GM3 (N-acetylneuraminic acid) in the following order of reactivity: > GT1b > GD3 > GD1a > GQ1b, but did not react with lactosylceramide, GM3 (N-glycolylneuraminic acid), GM2, GM1, GD2, GD1b, 9-o-Ac-GD3, SSEA-1 ($Le^X$) and sialylparagloboside. The antibody of the present invention which AKH11 cells produce, will be hereinafter referred to as AKH11 antibody.

The above measurement was conducted with reference to "Protein, Nucleic Acid and Enzyme", No. 31 Separate Edition, p. 183-192 (1987).

The following Examples were conducted in accordance with the following experimental textbooks unless otherwise specified:

(1) T. Maniatis, E. F. Fritsch, J. Sambrook: Molecular Cloning, Cold Spring Harbor Laboratory (USA)
(2) F. M. Ausabel, et al.: Current Protocols in Molecular Biology, John Wiley & Sons, Inc. (USA)

EXAMPLE 2

Preparation of a cDNA library using mRNA derived from AKH11 antibody-producing cells

The EBV-transformed cell line AKH11 obtained in Example 1 (1) was cultured at 37°C for 96 hours in a 20% FCS-RPMI culture medium. Then, 1 × $10^8$ AKH11 cells were subjected to homogeneous lysis by means of a 20-G injection needle in 3.5 ml of a 4M guanidinium isothiocyanate solution containing 20 mM sodium acetate, 0.1 mM DTT and 0.5% of N-lauryl sarcosine. The mixture thereby obtained was put on 1.5 ml of a 5.7 M cesium chloride solution (5.7M cesium chloride, 0.1 mM EDTA), followed by centrifugation at 18°C at 35,000 rpm for 21 hours to obtain a precipitate containing mRNA. This precipitate was dissolved in a 10 mM Tris-HCl (pH7.4) solution containing 5 mM EDTA and 1% of SDS and then precipitated with ethanol to obtain mRNA.

This mRNA was adsorbed on an oligo (dT) cellulose column and eluted with a 20 mM Tris-HCl (pH7.6) solution containing 0.5M NaCl, 1 mM EDTA and 0.1% of sodium lauryl sarcosinate to fractionate mRNA containing poly (A). This mRNA was further precipitated with ethanol and dissolved in a 10 mM Tris-HCl (pH8.0) solution containing 1 mM EDTA. Using this poly (A) mRNA as a template, cDNA was prepared by means of a cDNA synthesis kit (RPN1256Y, manufactured by Amersham). To this cDNA, EcoRI linker was added, and then it was inserted into pUC19 vector to obtain a cDNA library. The plasmid vector containing cyclized cDNA was infected to Escherichia coli JM109 (E. coli JM109 competent cells, prepared by Takara Shuzo)to obtain a cDNA library using E. coli JM109 as the host.

EXAMPLE 3

Isolation of a plasmid containing cDNA of AKH11 antibody and determination of the DNA sequence thereof

The above AKH11 cDNA library using E. coli JM109 was transferred on nylon filters (Hybond-N, manufactured by Amersham), and a replica filter was prepared by a pair of such nylon filters as master filters. E. coli on the replica filter was put on a filter paper (Whatman 3MM paper) wetted with a denaturing solution (0.5M NaOH, 1.5M NaCl) for 7 minutes, then on a filter paper (same as above) wetted with a neutralizing solution (1.5M NaCl, 0.5M Tris-HCl (pH7.4)) for 3 minutes and on a filter paper (same as above) wetted with the neutralizing solution afresh for 3 minutes, and further washed a 2 × SSC solution (0.06M NaCl, 6 mM sodium citrate), followed by UV irradiation (UV Stratalinker 1800 Model, manufactured by SCS) to fix the plasmid DNA on the filter.

On the other hand, oligonucleotide 5'-AATTCTCACAGGAGACGAGG-3' of SEQ ID NO:3 in the Sequence Listing complementing a part (the DNA sequence corresponding to the 33-52nd) of the CH1 domain of the DNA sequence of the human IgM antibody μ-chain gene already reported (T. H. Rabbitts, A. Forster, C. P. Milstein: Nucleic Acids Res., Vol. 9, p. 4509 (1981)) and oligonucleotide 5'-AGGCCAGCAGGGTCAGTAGC-3' of SEQ ID NO:4 in the Sequence Listing complementing a part (the DNA sequence corresponding to the 338-357th) of the CH4 domain, were prepared, and the two were used as probes for screening μ-chain cDNA. Further, in the same manner as above, oligonucleotide 5'-AAGATGAAGACAGATGGTGC-3' of SEQ ID NO:5 in the Sequence Listing complementing a part (the DNA sequence corresponding to the 345-364th) of the DNA sequence of the human IgM antibody κ-chain gene (P. A. Hieter, E. E. Max, J. G. Seidman, J. V. Maizel Jr., P. Leder: Cell, Vol. 22, p. 197 (1980)) and oligonucleotide 5'-ACTTCTCCCTCTAACACTCT-3' of SEQ ID NO: 6 in the Sequence Listing complementing a part (the DNA sequence corresponding to the 647-666th) of the DNA sequence, were prepared, and the two were used as probes for screening κ-chain cDNA. The 5'-terminals of these oligonucleotide probes were labeled with $^{32}P$ by means of T4 polynucleotide kinase and [γ-32P]ATP. The labeled probes were separately hybridized to replica filters having plasmid DNA fixed thereto.

The hybridizing reaction was conducted at 40°C for 16 hours in 10 ml of a solution comprising 5 × Denhardt's, 0.1% SDS, 10 μCi of the labeled probe and 5 × SSC (0.15M NaCl, 0.015M sodium citrate) having 100 μg/ml of a degenerated salmon sperm DNA. After the reaction, the filter was washed with a 6 × SSC solution (0.18M NaCl, 0.018M sodium citrate) containing 0.1% of SDS three times at room temperature for 30 minutes and twice at 43°C for 60 minutes (above-mentioned experimental textbook (1) Molecular Cloning p. 309). Radioautograms were taken from the washed filters, and cell lines reactive with two types of the probes, were selected by putting one on the other of the radioautograms of the pair of replica filters. By this method, E. Coli JM109 cell line reactive with the probes of the μ-chain gene portion and E. coli JM109 cell line reactive with the probes of the κ-chain gene portion were obtained.

From these cell lines, plasmid DNA was extracted by an alkali method (H. C. Birnboim, J. Doly: Nucleic Acids Res., Vol. 7, p. 1513 (1979)).

The plasmid DNA was digested with restriction endonuclease EcoRI (manufactured by Takara Shuzo) and fractionated by agarose gel electrophoresis. Then, DNA fragments in the agarose gel were transferred to nylon filters (Hybond-N, manufactured by Amersham). The filters were hybridized with the above oligonucleotide probes, whereby the DNA fragments reacted with the probes. Then, E. coli JM109 2-14 (the one reacted with the probes of the μ-chain gene portion) and E. coli JM109 K5-1-1 (the one reacted with the probes of the κ-chain gene portion) having plasmids and forming the maximum EcoRI DNA fragments (cDNA portions) were selected, respectively, from them. These strains were deposited as JMp2-14 and JMpK5-1-1, respectively, at the Fermentation Research Institute, Agency of Industrial Science and Technology (FERM BP3638 and FERM BP3639). Further, these strains contain p2-14 and pK5-1-1 as recombinant plasmids.

The DNA sequences of the cDNA portions of the plasmid DNAs (p2-14 and pK5-1-1) of E. coli JM109 2-14 and E. coli JM109 K5-1-1, were determined by a dideoxynucleotide chain termination method (J. Messing et al: Nucleic Acids Res., Vol. 9, p. 309 (1981)). As a result, the DNA sequence and the amino acid sequence of the V-region of the μ-chain of AKH11 antibody were determined in accordance with the above-mentioned literature (T. H. Rabbitts, A. Foster, C. P. Milstein: Nucleic Acids Res., Vol. 9, p. 4509-4524 (1981)) and shown by SEQ ID NO:1 in the Sequence Listing, and the DNA sequence and the amino acid sequence of the V-region of the κ-chain were determined in accordance with the above-mentioned literature (P. A. Hieter, E. E. Max, J. G. Seidman, J. V. Maizel Jr., P. Leder: Cell, Vol. 22, p. 197-207 (1980)) and shown by SED ID NO:2 in the Sequence Listing.

## EXAMPLE 4

### Cloning of 2-14Rc/CMV expression vector

Plasmid p2-14 prepared in Example 3 was digested with restriction endonucleases NcoI (manufactured by Takara Shuzo) and SalI (manufactured by Takara Shuzo), and a DNA fragment containing the μ-chain gene of AKH11 antibody was separated. To this DNA fragment, a Klenow fragment (manufactured by Takara Shuzo) was reacted to form a blunt end. Then, a synthesized linker was ligated by T4DNA ligase. The synthesized linker was prepared in such a manner that an oligonucleotide of 8 bases having a sequence represented by 5'-CTCTAAAG-3' and an oligonucleotide of 12 bases having a sequence complementing such an oligonucleotide and having a sequence of 5'-CACA-3' added to the 3'-side thereof, were prepared, and their 5'-termini were phosphorylated, respectively, with a polynucleotide kinase, followed by annealing the two. After deactivating the T4DNA ligase by heat treatment at 65°C for 10 minutes, the DNA fragment containing the μ-chain gene of AKH11 antibody having a linker ligated, was separated by agarose gel electrophoresis. On the other hand, plasmid pRc/CMV (manufactured by Invitrogen) was digested with restriction endonuclease Bst XI, and this ring-opened plasmid and the above DNA fragment containing the μ-chain gene of AKH11 antibody having the synthesized linker ligated, were mixed and reacted with T4DNA ligase to clone an expression vector 2-14Rc/CMV having the μ-chain gene of AKH11 antibody inserted downstream of the cytomegalovirus promoter.

## EXAMPLE 5

### Cloning of expression vector K5-1-1Rc/CMV

The plasmid pK5-1-1 prepared in Example 3 was digested with restriction endonuclease EcoRI, and a DNA fragment containing the κ-chain gene of AKH11 antibody was separated. To this DNA, a Klenow fragment was reacted to form a blunt end. Then, a synthesized linker (the same as in Example 4) was ligated by T4DNA ligase. After deactivating the T4DNA ligase by heat treatment at 65°C for 10 minutes, the DNA fragment containing the κ-chain gene of AKH11 antibody having the linker ligated, was separated by agarose gel electrophoresis.

On the other hand, plasmid pRc/CMV was digested with restriction endonuclease Bst XI, and this ring-opened plasmid and the above DNA fragment containing the $\kappa$-chain gene of AKH11 antibody having the synthesized linker ligated, were mixed and reacted with T4DNA ligase to clone an expression vector K5-1-1Rc/CMV having the $\kappa$-chain K5-1-1 gene of AKH11 antibody inserted downstream of the cytomegalovirus promoter.

EXAMPLE 6

Transfection of the expression vectors to mouse myeloma cell line XA653

Mouse myeloma cells XA653 (ATCC No. CRL-1580) were cultured in a 10% fetal calf serum-containing DMEM culture medium, and then the cells were transferred to a DMEM culture medium containing no serum. Then, 5 ml of the cell suspension having a cell density of $5 \times 10^5$ cells/ml was introduced into a dish having a diameter of 6 cm, and to the dish, a mixture comprising 5 $\mu$l of expression vector 2-14Rc/CMV (28.5 $\mu$g) DNA, 5 $\mu$l of expression vector K5-1-1Rc/CMV (18.5 $\mu$g) DNA, 20 $\mu$l of DEAE-dextran and 70 $\mu$l of TE (10 mM Tris-HCl (pH8.0), 1 mM EDTA) was added. Finally, chloroquine was added to a final concentration of 100 $\mu$M, followed by culturing for 4 hours. Then, the culture solution was changed to a 10% FCS-RPMI culture medium, followed by culturing for further 4 days. The culture solution was collected and recovered, and the biological activities were measured to determine whether or not a recombinant AKH11 antibody was produced by the transfection of the expression vectors.

EXAMPLE 7

Determination of the biological activities of the recombinant AKH11 antibody

The biological activities of the recombinant AKH11 antibody contained in the culture solution obtained in Example 6 were measured, whereby the amount of IgM of the recombinant AKH11 antibody produced was measured by an ELISA method (An enzyme linked immuno sorbent assay)

The measurement of the CDC activity was conducted by the method described in Example 1 (1). The results were as shown in Table 1.

Table 1

| Cell line | % lysis |
|---|---|
| MeWo | 41.2% |
| M12 | 43.8% |
| Human neuroblastoma (IMR-32) | 0% |

Then, the amount of IgM was measured by an ELISA method. Firstly, a 10 $\mu$g/ml solution of IgG fraction goat anti-human IgG (Fab fragment specific, No. 0201-0101, manufactured by Cappel) was adsorbed on a microplate with 96 wells in an amount of 100 $\mu$l/well at 4°C for 18 hours. Then, non-specific bonds were blocked by a borate buffered solution (pH8.0) containing 1 mg/ml of egg albumin (4°C, 18 hours). Then, a culture solution containing the recombinant AKH11 antibody was added in an amount of 50 $\mu$l/well and reacted at 37°C for one hour. Further, a 10 $\mu$g/ml peroxidase conjugated goat anti-human IgM ($\mu$-chain specific, No. 3601-0201, manufactured by Cappel) was added in an amount of 50 $\mu$l/well and reacted at 37°C for one hour. As an enzyme substrate, a substrate buffer solution (0.1M citric acid, 0.2M sodium phosphate dibasic, 0.005% hydrogen peroxide) containing 0.4 mg/ml of o-phenylenediamine was reacted in an amount of 100 $\mu$l/well, whereupon the absorbance at 450 nm was measured, and the amount of IgM was calculated. For the calculation of the amount of IgM, human IgM (No. 6001-1590, manufactured by Cappel) was used as a control. The amount of IgM in the culture solution containing the recombinant AKH11 antibody was 100 ng/ml.

From the above results, it is evident that a recombinant antibody having the same activities as AKH11 antibody can be produced by transfecting expression vector 2-14Rc/CMV and expression vector K5-1-1Rc/CMV to mouse myeloma cells XA653 which are basically of non-secretor.

The antibody of the present invention is a novel antibody capable of recognizing a common antigen of ganglioside GM3 (N-acetylneuraminic acid), GT1b, GD3, GD1a and GQ1b, and it shows a CDC activity against melanoma such as MeWo, M12, M14 or G-361, while it shows no substantial CDC activity against a human normal skin fibroblast, a human adenocarcinoma, a human neuroblastoma or a Vero cell. Thus, it specifically recognizes melanoma and has a CDC activity. Further, it is derived from human and thus has low antigenicity against human and no substantial side effects.

Further, using the gene of the antibody of the present invention, a coded antibody can be produced by gene manipulation.

Accordingly, the antibody of the present invention is useful as a diagnostic or therapeutic composition for melanoma. It is also useful as a reagent for the study of the relation between the sugar chain and the carcinogenetic mechanisms or the role in vivo of the sugar chain.

SEQUENCE LISTING

SEQ ID NO:1:

SEQUENCE CHARACTERISTICS:
LENGTH: 497 base pairs
TYPE: nucleic acid
STRANDEDNESS:
TOPOLOGY: linear

MOLECULE TYPE: cDNA to mRNA

ORIGINAL SOURCE:
ORGANISM: Homo sapience
CELL TYPE: B cell

IMMEDIATE SOURCE:
CLONE: AKH 11

FEATURE:
KEY: sig peptide
LOCATION: 66..122
IDENTIFICATION METHOD: S

FEATURE:
KEY: mat peptide
LOCATION: 123..497
IDENTIFICATION METHOD: S

SEQUENCE DESCRIPTION: SEQ ID NO:1:

```
CCCAGCCC TGGGATTTTC AGGTGTTTTC ATTTGGTGAT CAGGACTGAG CAGAGAGAAC      60

TCACC ATG GAG TTT GGG CTG CCC TGG CTT TTT CTT GTG GCT ATT TTA      107
      Met Glu Phe Gly Leu Pro Trp Leu Phe Leu Val Ala Ile Leu
                      -15                 -10

AAA GGT GTC CAG TGT GAG GTG CAG CTG TTG GAA TCT GGG GGA GGC TTG      155
Lys Gly Val Gln Cys Glu Val Gln Leu Leu Glu Ser Gly Gly Gly Leu
-5                  1               5                   10

GTA CAG CCT GGG GGG TCC CTG AGA CTC TCC TGT GCA GCC TCT GGA TTC      203
Val Gln Pro Gly Gly Ser Leu Arg Leu Ser Cys Ala Ala Ser Gly Phe
            15                  20                  25

ACC TTT AGC AGC GAT GTC ATG AGC TGG GTC CGC CAG GCT CCA GGG AAG      251
Thr Phe Ser Ser Asp Val Met Ser Trp Val Arg Gln Ala Pro Gly Lys
        30                  35                  40

GGG CTG GAG TGG GTC TCA AGT ATT AAT GGT AGA GGT GGA ATT TCG TAC      299
Gly Leu Glu Trp Val Ser Ser Ile Asn Gly Arg Gly Gly Ile Ser Tyr
    45                  50                  55

TAC GCA GAC TCC GTG AAG GGC CGG TTC ACC ATC TCC AGA GAC AAT TCG      347
Tyr Ala Asp Ser Val Lys Gly Arg Phe Thr Ile Ser Arg Asp Asn Ser
60                  65                  70                  75
```

```
AAG AAC ACG CTG TAT CTG CAA ACG AAC AGC CTG AGA GCC GAG GAC ACG    395
Lys Asn Thr Leu Tyr Leu Gln Thr Asn Ser Leu Arg Ala Glu Asp Thr
                80                  85                  90

GCC ATA TAT TAC TGT GCG AAA TGT AGC GGC GAG ATA CGA CTA TGC TTT    443
Ala Ile Tyr Tyr Cys Ala Lys Cys Ser Gly Glu Ile Arg Leu Cys Phe
            95                  100                 105

TGG AGC CAC TGG GGC CAG GGA ACC CTG GTC ACC GTC TCC TCA GGG AGT    491
Trp Ser His Trp Gly Gln Gly Thr Leu Val Thr Val Ser Ser Gly Ser
        110                 115                 120

CGA TCC                                                            497
Arg Ser
    125
```

SEQ ID NO:2:

SEQUENCE CHARACTERISTICS:
LENGTH: 407 base pairs
TYPE: nucleic acid
STRANDEDNESS:
TOPOLOGY: linear

MOLECULE TYPE: cDNA to m RNA

ORIGINAL SOURCE:
ORGANISM: Homo sapience
CELL TYPE: B cell

IMMEDIATE SOURCE:
CLONE: AKH 11

FEATURE:
KEY: sig peptide
LOCATION: 6..65
IDENTIFICATION METHOD: S

FEATURE:
KEY: mat peptide
LOCATION: 66..407
IDENTIFICATION METHOD: S

SEQUENCE DESCRIPTION: SEQ ID NO:2:

```
GCAAG ATG GTG TTG CAG ACC CAG GTC TTC ATT TCT CTG TTG CTC TGG      47
      Met Val Leu Gln Thr Gln Val Phe Ile Ser Leu Leu Leu Trp
      -20                 -15                 -10

ATC TCT GGT GCC TAC GGG GAC ATC GTG ATG ACC CAG TCT CCA GAC TCC    95
Ile Ser Gly Ala Tyr Gly Asp Ile Val Met Thr Gln Ser Pro Asp Ser
    -5                  1                   5                   10

CTG GCT GTG TCT CTG GGC GAG AGG GCC ACC ATC AAC TGC AAG TCC AGC   143
Leu Ala Val Ser Leu Gly Glu Arg Ala Thr Ile Asn Cys Lys Ser Ser
                15                  20                  25
```

```
CAG AGT GTT TTA TAC AGC TCC AAC AAC AAG AAC TAC TTA GCT TGG TAC     191
Gln Ser Val Leu Tyr Ser Ser Asn Asn Lys Asn Tyr Leu Ala Trp Tyr
            30                  35                  40

CAG CAG AAA CCG GGA CAG CCT CCT AGG CTG CTG ATT TCC TGG GCA TTT     239
Gln Gln Lys Pro Gly Gln Pro Pro Arg Leu Leu Ile Ser Trp Ala Phe
        45                  50                  55

ACC CGG GAA TCC GGG GTC CCT GAC CGA TTC AGT GGC AGC GGG TCG GGG     287
Thr Arg Glu Ser Gly Val Pro Asp Arg Phe Ser Gly Ser Gly Ser Gly
    60                  65                  70

ACA GAT TTC ACT CTC ACC ATC AAC AGC CTG CAG GCT GAT GAT GTG GCA     335
Thr Asp Phe Thr Leu Thr Ile Asn Ser Leu Gln Ala Asp Asp Val Ala
75                  80                  85                  90

GTT TAT TAC TGT CAG CAA TAT TAT AAT ACT CCT CCT CTG TTC GGC CAA     383
Val Tyr Tyr Cys Gln Gln Tyr Tyr Asn Thr Pro Pro Leu Phe Gly Gln
                95                 100                 105

GGG ACC AAG GTG GAA ATC AAA CGA                                     407
Gly Thr Lys Val Glu Ile Lys Arg
           110
```

SEQ ID NO:3:

SEQUENCE CHARACTERISTICS:
LENGTH: 20 base pairs
TYPE: nucleic acid
STRANDEDNESS: single
TOPOLOGY: linear

MOLECULE TYPE: Genomic DNA

SEQUENCE DESCRIPTION: SEQ ID NO:3:

AATTCTCACA GGAGACGAGG 20

SEQ ID NO:4:

SEQUENCE CHARACTERISTICS:
LENGTH: 20 base pairs
TYPE: nucleic acid
STRANDEDNESS: single
TOPOLOGY: linear

MOLECULE TYPE: Genomic DNA

SEQUENCE DESCRIPTION: SEQ ID NO:4:

AGGCCAGCAG GGTCAGTAGC 20

SEQ ID NO:5:

SEQUENCE CHARACTERISTICS:
LENGTH: 20 base pairs
TYPE: nucleic acid
STRANDEDNESS: single
TOPOLOGY: linear

MOLECULE TYPE: Genomic DNA

SEQUENCE DESCRIPTION: SEQ ID NO:5:

AAGATGAAGA CAGATGGTGC 20

SEQ ID NO:6:

SEQUENCE CHARACTERISTICS:
LENGTH: 20 base pairs
TYPE: nucleic acid
STRANDEDNESS: single
TOPOLOGY: linear

MOLECULE TYPE: Genomic DNA

SEQUENCE DESCRIPTION: SEQ ID NO:6:

ACTTCTCCCT CTAACACTCT 20

## Claims

**1.** A human monoclonal antibody with the class of immunoglobulin being IgM and with the type of L-chain being *x* and capable of recognizing a common antigen of ganglioside GM3 (N-acetylneuraminic acid), GT1b, GD3, GD1a and GQ1b present on the surface of a melanoma cell of MeWo, M12, M14 or G-361.

**2.** The human monoclonal antibody according to Claim 1, which shows a complement dependent cytotoxic activity against the melanoma cell of MeWo, M12, M14 or G-361 having the common antigen, but shows no substantial complement dependent cytotoxic activity against a human normal skin fibroblast, a human a denocarcinoma, a human neuroblastoma or a Vero cell.

**3.** The human monoclonal antibody according to Claim 1, which is AKH11 antibody.

**4.** A process for producing a human monoclonal antibody, which comprises recovering B lymphocytes from the peripheral blood of a melanoma patient, transforming them by Epstein-Barr virus to establish a human cell line capable of producing the human monoclonal antibody as defined in Claim 1, culturing the cell line in a culture medium, followed by recovery of the antibody.

**5.** A diagnostic or therapeutic composition for melanoma, which contains the human monoclonal antibody as defined in Claim 4 in an amount sufficient for the diagnosis or therapy of melanoma.

**6.** A human cell line transformed by Epstein-Barr virus, which is capable of producing the human monoclonal antibody as defined in Claim 1.

**7.** An antibody gene containing a gene encoding the human monoclonal antibody as defined in Claim 1 or 2.

**8.** A gene containing a gene encoding an amino acid sequence of the 1st Glu to the 117th Val as shown by SEQ ID NO:1 in the Sequence Listing.

9. A gene containing a DNA sequence of the 123-125th GAG to the 471-473rd GTC as shown by SEQ ID NO:2 in the Sequence Listing.

10. A gene containing a gene encoding an amino acid sequence of the 1st Asp to the 114th Arg as shown by SEQ ID NO:2 in the Sequence Listing.

11. A gene containing a DNA sequence of the 66-68th GAC to the 405-407th CGA as shown by SEQ ID NO:2 in the Sequence Listing.

12. A recombinant plasmid containing the gene as defined in Claim 7, 8, 9, 10 or 11.

13. The recombinant plasmid according to Claim 12, which is p2-14 or pK5-1-1.

14. The recombinant plasmid according to Claim 12, wherein the expression vector is 2-14Rc/CMV or K5-1-1Rc/CMV.

15. A recombinant cell containing the recombinant plasmid as defined in Claim 12, 13 or 14.

16. The recombinant cell according to Claim 15, wherein the host cell is Escherichia coli or a mammalian cell.

17. The recombinant cell according to Claim 15, which is JMp2-14 or JMpK5-1-1 (FERM BP-3638 or FERM BP-3639).

18. The recombinant cell according to Claim 15, which contains an expression vector 2-14Rc/CMV or K5-1-1Rc/CMV.

19. A process for producing a human monoclonal antibody, which comprises culturing the recombinant cell as defined in Claim 15, 16 or 18 in a culture medium, followed by recovery of the antibody.

20. A human monoclonal antibody, wherein the V-region of H-chain contains an amino acid sequence of the 1st Glu to the 117th Val as shown by SEQ ID NO:1 in the Sequence Listing, and the V-region of L-chain contains an amino acid sequence of the 1st Asp to the 114th Arg as shown by SEQ ID NO:2 in the Sequence Listing.

21. A diagnostic or therapeutic composition for melanoma, which contains the human monoclonal antibody as defined in Claim 20 in an amount sufficient for the diagnosis or therapy of melanoma.

# FIGURE 1

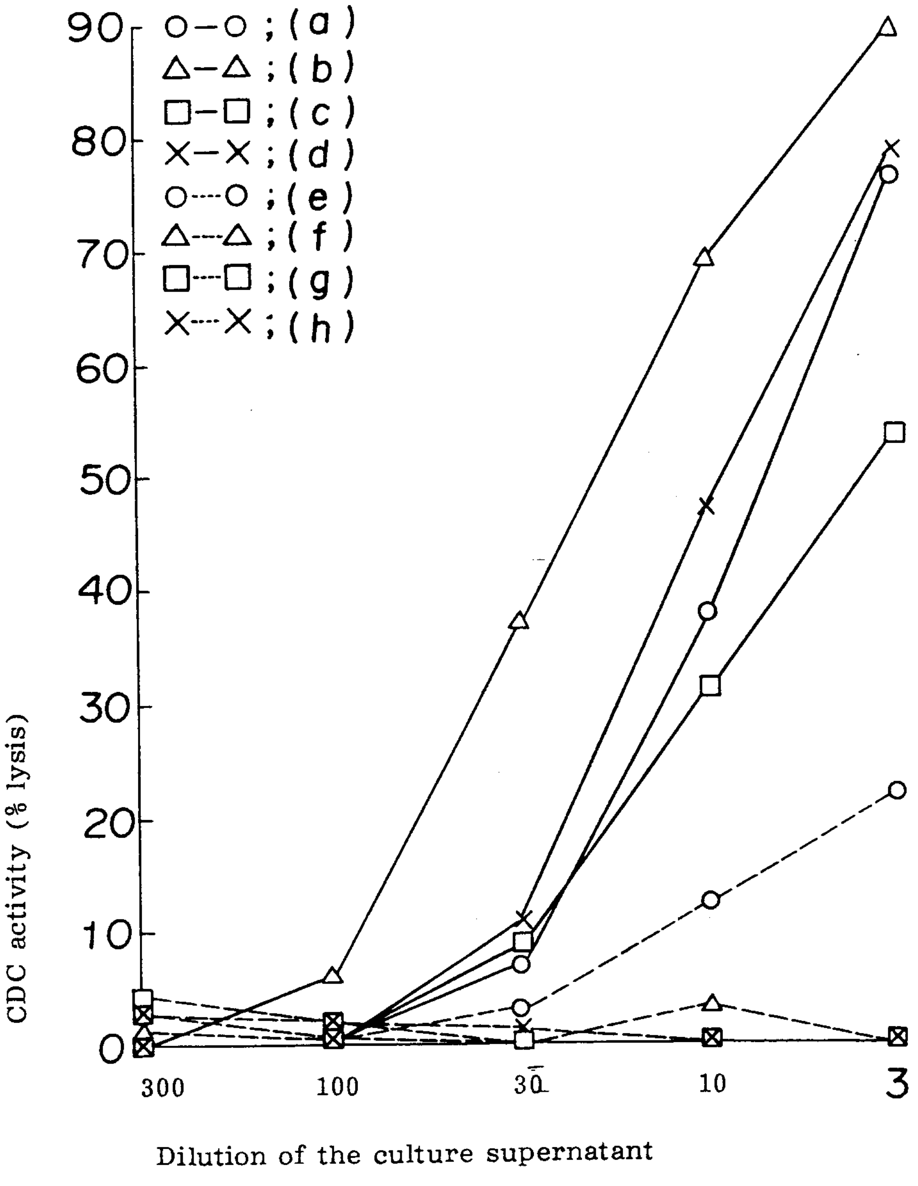
o–o ; (a)
△–△ ; (b)
□–□ ; (c)
×–× ; (d)
o----o ; (e)
△----△ ; (f)
□----□ ; (g)
×----× ; (h)
CDC activity (% lysis)
90
80
70
60
50
40
30
20
10
0
300
100
30
10
3
Dilution of the culture supernatant

European Patent Office

# EUROPEAN SEARCH REPORT

Application Number

EP 91121727.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | US - A - 5 006 470 (YAMAGUCHI et al.) * Totality * -- | 1,4,5, 7,21 | C 07 K 15/28<br>C 12 N 15/13<br>C 12 N 5/22<br>C 12 N 15/11<br>C 12 N 5/12<br>G 01 N 33/577<br>A 61 K 39/395 |
| X | EP - A - 0 381 310 (THE BIOMEMBRANE INSTITUTE) * Totality, especially claims * -- | 1,4,5, 7,21 | |
| A | CHEMICAL ABSTRACTS, vol. 106, no. 7, February 16, 1987, Columbus, Ohio, USA L. FREDDO et al. "Monoclonal anti-DNA IgMk in neutropathy binds to myelin and to a conformational epitope formed by phosphatidic acid and gangliosides". pages 481-482, abstract-no. 48 354c & J. Immunol. 1986, 137(12), 3821-5 -- | 1,5,21 | **TECHNICAL FIELDS SEARCHED (Int. Cl.5)** |
| A | CHEMICAL ABSTRACTS, vol. 97, no. 21, November 22, 1982, Columbus, Ohio, USA E. NUDELMAN et al. "Characterization of a human melanoma-associated ganglioside antigen defined by a monoclonal antibody, 4.2". page 602, abstract-no. 179 891u & J. Biol. Chem. 1982, 257(21), 12752-6 ---- | 1,5,21 | C 07 K<br>C 12 N<br>C 12 P<br>G 01 N 33/00<br>A 61 K |

The present search report has been drawn up for all claims

| Place of search | Date of completion of the search | Examiner |
|---|---|---|
| VIENNA | 27-03-1992 | SCHNASS |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document